(19) Europäisches Patentamt
European Patent Office
Office européen des brevets

(11) Publication number : 0 482 948 A1

# (12) EUROPEAN PATENT APPLICATION

(21) Application number : 91309891.9

(22) Date of filing : 25.10.91

(51) Int. Cl.$^5$ : **A61K 9/14**, A61K 9/18, A61K 47/38, A61K 31/495

(30) Priority : 26.10.90 JP 290166/90

(43) Date of publication of application : 29.04.92 Bulletin 92/18

(84) Designated Contracting States : CH DE ES FR GB IT LI

(71) Applicant : TOYO JOZO KABUSHIKI KAISHA
632-1 Mifuku Ohito-cho
Tagata-gun Shizuoka-ken (JP)

(72) Inventor : Hasegawa, Masaki
230-16, Makino-go, Shujenji-cho
Tagata-gun, Shizuoka (JP)
Inventor : Sumita, Ikuo
379-11, Mifuku, Ohito-cho
Tagata-gun, Shizuoka (JP)

(74) Representative : Hayes, Adrian Chetwynd et al
J. A. Kemp & Co., 14 South Square, Gray's Inn
London WC1R 5LX (GB)

## (54) A medicament composition containing 6-substituted alkoxy quinoxaline derivative and its production.

(57) A composition which comprises an amorphous mixture of a compound of formula (I)

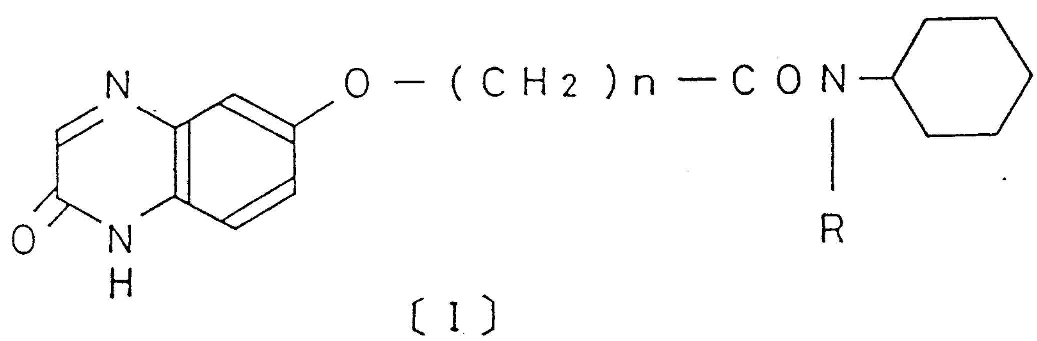

( I )

wherein R is methyl or ethyl and n is 3 or 4, and a hydroxypropyl cellulose, or the compound of formula (I) in amorphous form and a hydroxypropyl cellulose.

EP 0 482 948 A1

This invention relates to a medicament composition with improved water-solubility and which comprises a slightly soluble compound of formula (I):

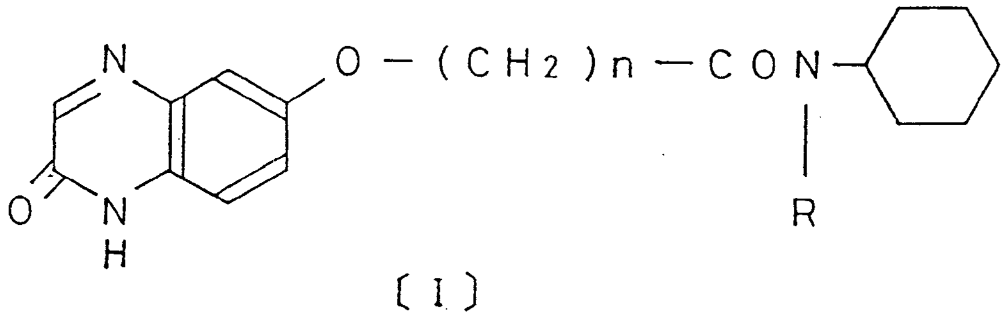

( I )

wherein R is methyl or ethyl and n is 3 or 4, and process for the production thereof.

The above 6-substituted alkoxy-2-oxo-1,2-dihydroquinoxaline derivative of formula (I) has platelet aggregation inhibitory action and/or cyclic-AMP phosphodiesterase inhibitory action. It is a useful medicament for treatment of thrombosis and for improvement of blood circulation (JP-A-2-76860).

The compound of formula (I) has extremely low solubility in water. Conventional means for improving its solubility in water have failed to increase the solubility. A method for increasing the water solubility slightly by adding cyclodextrins is known (JP-A-2-221223). However the method cannot provide a sufficient improvement in the water solubility.

In general, when a slightly soluble medicament is administered orally, a pharmacological effect is not expected due to poor absorption in the gastrointestinal tract. It is well known that an increase in the solubility of a slightly soluble medicament makes its bioavailability high and maintains the pharmacological effectiveness, so various ways of improving the solubility of slightly soluble pharmaceutical compounds have been reported as follows:

(1) β-1,4-glucan is mixed with a slightly soluble medicament and pulverized to an amorphous state (JP-B-54-295659);

(2) A soluble protein such as gelatin and a hydrophilic polymer such as methyl cellulose, hydroxypropyl cellulose or poly (vinyl-pyrrolidone) are mixed with a slightly soluble medicament and pulverized to an amorphous state (JP-B-64-6174);

(3) A slightly soluble medicament and quinoxaline are mixed with pulverization until an amorphous state is achieved (JP-B-63-28414);

(4) A slightly soluble medicament is amorphously dispersed into a mixture of poly (vinyl-polypyrrolidone) and a hydrophilic polymer such as methyl cellulose (JP-B-63-60010);

(5) A slightly soluble medicament and a hydrophilic polymer such as poly (vinyl-pyrrolidone) are kneaded by a roller mixer under heating at ambient temperature or at a temperature under the melting point of the medicament (JP-A-60-190723);

(6) A slightly soluble medicament and a polymer such as hydroxypropyl cellulose are dissolved in organic solvent, powdered granules which are insoluble in the solvent are added with kneading, and then the solvent is removed (JP-A-61-249914);

(7) A mixture of a slightly soluble medicament and partial α-starch is spray dried (JP-A-63-101333);

(8) A slightly soluble medicament is uniformly mixed with a water swelling polymer such as hydroxypropyl cellulose, sodium carboxymethyl starch and partial α-starch, a polymer which is soluble in an organic solvent and soluble in a buffer solution of pH 1 to 9 such as hydroxypropyl cellulose, sodium carboxymethyl cellulose, hydroxypropyl methyl cellulose and poly (vinyl-pyrrolidone), and/or a monovalent metallic salt polymer which is soluble in water (JP-A-1-156909); and

(9) A solvent is rapidly removed from a solution containing a dissolved slightly soluble medicament, a hydrophilic polymer such as poly (vinyl-pyrrolidone), crystalline cellulose and cross-linked carboxymethyl cellulose and/or hydrophilic inorganic fine particles in an organic solvent, or a suspension suspended in the same (JP-A-2-49720).

Since the compound of formula (I) has extremely low solubility in water, oral preparations thereof have low bioavailability due to bad dissolution in the gastrointestinal tract. In order to improve the dissolution a composition containing cyclodextrins has been prepared. However the solubility in water is still not improved.

We have tried to overcome the above disadvantage and tried to improve the solubility of the compound of formula (I). The prior arts such as (1) to (4) hereinabove defined, i.e. methods for preparing an amorphous mixture by mixed grinding of a slightly soluble medicament and a polymer in a mill such as ball-mill, have a number of disadvantages in that they lack simplicity due to requiring several tens of hours for achieving an amorphous state and cause a change in quality. Furthermore the prior arts (5) to (8) do not provide an improved solubility of the compound of formula (I). The prior art (9) was tried using the polymer described in the reference. However there was less improved effect of increasing the solubility of the compound of formula (I), namely supersaturation did not continue and the effect, i.e. the increase in solubility, was temporary. Thus the prior methods do not provide an improvement in the solubility of the slightly soluble medicament, namely they may depend on the nature of the medicament, its properties, the kind of polymer used and the method of treatment.

The present invention provides a composition which comprises an amorphous mixture of a compound of formula (I) as defined above and a hydroxypropyl cellulose, or the compound of formula (I) in amorphous form and a hydroxypropyl cellulose.

The present invention also provides a process for the production of a composition as defined above which comprises adding the hydroxypropyl cellulose into the compound of formula (I) in the amorphous state or converting a mixture of the compound of formula (I) and hydroxypropyl cellulose to the amorphous state.

The present invention additionally provides a composition as defined above for use in a method of treatment of the human or animal body by therapy, in particular for use in a method of treatment of thrombosis or for use in improving the blood circulation.

The present invention further provides use of a composition as defined above in the manufacture of a medicament for the treatment of thrombosis or for improving the blood circulation.

The composition of the present invention remarkably improves the solubility of the compound of formula (I). We have also found that among the polymers which could be used, only hydroxypropyl cellulose is effective for improving the solubility; other polymers have almost no effect. The composition obtained by drying the compound of formula (I) together with a hydroxypropyl cellulose by spray-drying or fluidized bed granulation drying provides remarkably improved solubility of the compound of formula (I). Also we have found that a composition which has a continuous supersaturation effect can be effectively produced within short time.

Brief explanation of the Drawings:

Fig. 1: Solubility curve of the medicament compositions 3 - 5 ;
Fig. 2: Solubility curve of the medicament compositions 1 - 2, their starting compound A and amorphous compound A ;
Fig. 3: Solubility curve of the medicament compositions 6 - 9 ;
Fig. 4: Solubility curve of the control compositions 1 - 3 ;
Fig. 5: X-ray diffraction pattern of the starting compound A ;
Fig. 6: X-ray diffraction pattern of the medicament composition 3 ;
Fig. 7: X-ray diffraction pattern of the control composition 1 ;
Fig. 8: Solubility curve of the medicament compositions 10 and its starting compound D ;
Fig. 9: Solubility curve of the control compositions 4 - 6 ;
Fig. 10: Solubility curve of the medicament compositions 11 - 13 ;
Fig. 11: Solubility curve of the medicament compositions 14 - 17 ;
Fig. 12: Solubility curve of the medicament compositions 18 - 19 ;
Fig. 13: Solubility curve of the control compositions 7 - 11 ; and
Fig. 14: Solubility curve of the control composition 20.

Detailed explanation of the Invention

The present invention relates to a medicament composition having substantially improved water-solubility of a pharmaceutical compound of the formula [ I ] comprising an amorphous composition of the mixture consisting of the said compound of the formula [ I ] and hydroxypropyl cellulose, or a composition of the mixture consisting of an amorphous compound [ I ] and a hydroxypropyl cellulose, and a process for production of the said composition.

Further the preset invention pertains to a medicament composition having substantially improved water-solubility of a pharmaceutical compound of the formula [ I ] comprising an amorphous composition of the mixture consisting of the said compound of the formula [ I ] , hydroxypropyl cellulose and stabilizing agents for amorphous state and process of production of the said composition.

Examples of the pharmaceutical compound [ I ] are 6-[3-(N-cyclohexyl-N-methyl-aminocarbonyl) propoxy] - 2 -oxo- 1, 2-dihydroquinoxaline (hereinafter designates as compound A), 6- [3-(N-cyclohexyl-N ethyl-aminocarbonyl) propoxy] - 2 - oxo-1,2-dihydroquinoxaline (hereinafter designates as compound B), 6- [4-(N-cyclohexyl-N-methyl-aminocarbonyl) butoxy]-2-oxo-1, 2-dihydroquinoxaline (hereinafter designates as compound C), and 6- [4-(N-cyclohexyl-N-ethyl-aminocarbonyl) butoxy ] -2-oxo-1, 2-dihydroquinoxaline (hereinafter designates as compound D). These compounds are known compound and their production is disclosed in Japan. Patent Unexam. Publ. No. 2-76860.

Preparation of the present medicament composition can be performed at first by dissolving the pharmaceutical compound [ I ] in a suitable amount of volatile organic solvent. Organic solvent are not limited if the compound [ I ] can be dissolved and it is volatile solvent, and are exemplified as alcohols, ketons, ethers or hydrocarbon chlorides. Among the methanol and methylene chloride are preferable. Concentration of the compound [ I ] is not limited but is usually 1 - 2% to approximately 10%.

The solution obtained hereinabove is rapidly dried to prepare amorphous mixture. The drying is effectively performed by rapid drying means with spray-drying or fluidized bed granulation drying. In the fluidized bed granulation drying, a solution of the compound [ I ] can be sprayed on a carrier such as lactose and starch in fluidized bed granulation drying machine.

A temporary improvement of solubility of the compound [ I ] can only be achieved by the above preparation of amorphous compound.

The medicament composition of the present invention can be obtained by adding hydroxypropyl cellulose (hereinafter designates as HPC) in the above amorphous compound [ I ].

Amount of HPC can be approx. over 0.25 part by weight and the upper limit thereof can be approx. 3 parts by weight for convenience of formulation procedure. Any grade of HPC which is 4 grade of SL, L, M and H according to its viscosity, can be used, and among these L is most preferable.

In the present invention, the pharmaceutical compound [ I ] can be dissolved together with HPC in an organic solvent for preparation of the medicament composition of the present invention. HPC can further be added to amorphous mixture of the pharmaceutical compound [ I ] and HPC. Total amount of HPC used in a mixture of the pharmaceutical compound [ I ] and HPC can be the same level of amount of HPC used in the aforementioned drying procedure.

In the medicament composition of the present invention, much higher level of amorphous state is preferable, and so formulation of highest grade amorphous compound or mixture is most preferable.

The thus prepared medicament composition of the present invention can provide an improved solubility of the pharmaceutical compound [ I ] in water, moreover supersaturation phenomenon can be continuously maintained for 120 - 180 minutes. The continuous maintaining supersaturation means that crystal-lization of the pharmaceutical compound [ I ] is prevented for long term, therefore absorption of almost water insoluble pharmaceutical compound such as the compound [ I ] in gastrointestinal tract can be markedly improved.

The above medicament composition of the present invention maintains in amorphous state for long term strage, however under severe long term strage at room temperature condition, for example at 40 °C and 75 % R.H. increase of crystallinity in amorphous compound is observed and solubility in water is decreased.

It is preferable to maintain stability of amorphous state that one or more stabilizing agent for amorphous state is dissolved together with the pharmaceutical compound [ I ] and HPC when dissolving in organic solvent, then preparing amorphous mixture by drying process hereinbefore explained.

Examples of stabilizing agent for amorphous state are hydroxypropyl methyl cellulose, hydorxypropyl methyl cellulose phthalate, ethyl cellulose, aminoalkyl methacrylate copolymer, methacrylate copolymer, polyvinylacetal diethylamino acetate or bile acid or its non-toxic salt. Amount of stabilizing agent for amorphous state to be used is preferably over approx. 0.25 part by weight, and its upper limit is preferably approx. 3 parts when considering for formulation.

Example of hydroxypropyl methyl cellulose is TC-5 (Shi-netsu Chem. Co.). Example of hydroxypropyl methyl cellulose phthalate is HP-50 and HP-55 (Shinetsu Chem. Co.). Example of ethyl cellulose is EC (Dow Chem. Corp.). Example of aminoalkylmethacrylate copolymer is Eudragit E and Eudragit RS (Rhom &Haas Co.). Example of methacrylate copolymer is Eudragit L and Eudragit S (Rhom & Haas Co.). Example of polyvinyl acetaldiethylamino acetate is AEA (Sankyo Co.). Example of bileacid is dehydrocholate and deoxycholate.

The thus prepared medicament composition of the present invention containing the amorphous mixture consisting of the pharmaceutical compound [ I ], HPC and stabilizing agent for amorphous state has improved solubility in water for the pharmaceutical compound [ I ] and moreover improved stability in amorphous state for the medicament composition under severe condition of long term storage.

The medicament composition of the present invention can be prepared for oral preparation, for example tablet, capsule, granule and syrup, by means of known method for formulation together with additive such as diluent, disintegrator, lubricant or stabilizer.

Effect of the Invention

Solubility in water at 37°C and degree in amorphous state measured by X-ray diffracton method of the medicament composition of the present invention and the control prepared by methods of referential examples are shown as follows.

Solubility curves of the medicament composition 3 ( -□- ), the medicament composition 4 (-+- ) and the medicament composition 5 (-◊- ) are shown in Fig. 1, in which good solubility curves are found and the continued supersaturation phenomenon is observed.

In Fig. 6, X-ray diffraction pattern of the medicament composition 3 of the present invention is shown wherein marked increase in amorphous state is observed as compared with a starting compound A (passed through 100 mesh sieve)(Fig. 5).

Solubility curves of the medicament composition 1 ( · · ◊ · · ), the medicament composition 2 ( --△-- ), a starting material ( -□- ) and the amorphous compound A ( -X- ) are shown in Fig. 2, in which good solubility of the medicament compositions 1 and 2 are found as compared with that of the compoud A.

Solubility curves of the medicament composition 6 ( -□- ), the medicament composition 7 ( · · + · · ), the medicament composition 8 ( · · ◊ · · ) and the medicament composition 9 ( --△-- ) are shown in Fig. 3, in which equivalent level of the effects are observed. Solubility curves of the control compound 1 (--△--), the control compound 2 ( -□- ) and the control compound 3 ( · · + · · ) are shown in Fig. 4, in which continuous solubility is observed for the control compound 1 with low value. Primary effects in solubility of the control compounds 2 and 3 are observed, however the effects are temporary. As shown the X-ray diffraction pattern of the control compound 1 in Fig. 7, lower level of amorphous state is observed as compared with the medicament composition 3.

Solubility curves of the medicament composition 10 ( · · + · · ) and the starting compound D ( -□- ) are shown in Fig. 8, in which it is found that a solubility of the medicament composition 10 is markedly increased as compared with the non-treated starting material. Solubility curves of the control compound 4 ( -□- ), the control compound 5 ( · · + · · ) and the control compound 6 ( · · ◊ · · ) are shown in Fig. 9, in which it can be found that 2 - 4 times increase in solubility are observed as compared with non-treated starting compounds.

As it can be seen hereinabove, the medicament composition of the present invention has improved solubility and continued supersaturation. In the process for preparation of the medicament composition of the present invention, amorphous pharmaceutical compound [ I ] is prepared and is mixed with the carrier selected from specific hydroxypropyl cellulose to produce the composition in which solubility of slightly soluble pharmaceutical compound is improved. The medicament composition of the present invention can be prepared without changing the property of the compound by simple production process which provides improved effect in dissolution with continued supersaturation phenomenon.

Further solubility curves of the medicament compositions 11-19 containing amorphous mixture consisting of the pharmaceutical compound [ I ], HPC and stabilizing agent for amorphous state are shown in Fig. 10 - Fig. 12, in which markedly increased solubility is observed as compared with that of the starting compound A and amorphous compound A (Fig. 2). From that result, it can be seen that not only markedly improved effect in dissolution for the pharmaceutical compound [ I ] is observed with or without adding stabilizing agents for amorphous state but also continuous supersaturation phenomenon is provided.

In Fig. 13, solubility curves of the control composition 7 - 11 which are prepared by spray-drying the pharmaceutical compound [ I ] and stabilizing agent for amorphous state without adding HPC, are shown. Markedly increased solubility as is observed in the medicament composition containing HPC is not observed.

The medicament compositions 2 and those of 11 - 19 are kept in strage with severe condition at 40 °C under humidity at 75 %, and stability in amorphous state of each medicament composition is measured. Results are shown in Table 1, in which a solubility of the medicament composition 2 without adding stabilizing agent for amorphous state is markedly decreased as compared with its initial preparation stage after strage at severe condition. On the contrary the solubility of the medicament compositions 11 - 19 with adding stabilizing agent for amorphous state is markedly improved and kept in amorphous state as compared with that of the medicament composition 2.

Stability test for amorphous state using the control compounds 12 and 13 in which stabilizing agent for amorphous state is replaced by vinyl acetate resin (Tanabe Seiyaku Co., food additive) and sucrose fatty acid ester (Daiichi Kogyo Seiyaku Co., DK-ester F 160), shows marked decrease in solubility after strage in severe condition as compared with that of initial stage. From that result, vinyl acetate resin and sucrose fatty acid ester have no effect as stabilizing agent for amorphous state.

As can be seen from the above, the medicament composition using stabilizing agent for amorphous state keeps its amorphous state for long term strage under severe condition.

## Examples

Following examples illustrate the present invention but are not construed as limiting.

### Example 1

A compound A (2 g) dissolved in methylene chloride (200 ml) was spray-dried using spray-drying machine (YAMATO Co., Type Pulvis GB22)(inlet temperature 70°C, outlet temperature 45 °C) to obtain amorphous powder of compound A. The powder (1 part by weight) was mixed with HPC-L (1 part by weight) in V-type mixer for 15 minutes to prepare the medicament composition 1.

### Example 2

A compound A (2 g) and HPC-L (2 g) were dissolved in methylene chloride (200ml), and the solution was spray-dried using spray-drying machine (inlet temperature 70 °C, outlet temperature 45 °C) to obtain the medicament composition 2.

### Example 3

In example 1, HPC-L was replaced by HPC-SL to obtain the medicament composition 3.

### Example 4

In example 2, HPC-L was replaced by HPC-M and HPC-H, respectively, to obtain the medicament compositions 4 and 5.

### Example 5

In example 2, amount of HPC-L (1 part by weight) was replaced by that of each 0.25, 0.5, 2 and 3 parts by weight, respectively, and treated as same as of in the example to obtain the medicament compositions 6, 7, 8 and 9.

### Referential example 1

A compound A (5 g) and HPC (5 g) were put into ball mill (volume 3.6 lit., balls 1 kg) and mixed to pulveriled for 12 hours to obtain the control medicament 1.

### Referential example 2

A compound A (2 g) and poly (vinyl pyrrolidone)(K-30, 2g) dissolved in methylene chloride (200 ml) and the solution was spray-dried according to the method described in example 2 to obtain the control medicament 2.

### Referential example 3

In referential example 2- poly(vinyl pyrrolidone)(K-30) was replaced by polyethylene glycol (6000) to obtain the control medicament 3.

### Example 6

In example 2 the compound A was replaced by the compound D to obtain the medicament composition 10.

### Referential example 4

A compound D (2 g) and HPC-L (2 g) were put into ball mill and mixed to pulveriled for 12 hours to obtain the control medicament 4.

Referential example 5

In example 2, pharmaceutical compound A was replaced by the compound D and HPC-L was replaced by poly (vinyl pyrrolidone)(K-30) and polyethylene glycol (6000), respectively, to obtain the control medicaments 5 and 6.

Example 7

In example 1, the compound A was replaced by the compounds B and C, respectively, to obtain the medicament compositions. These medicament compositions were shown improved solubility in water as same as of the medicament compositon 1.

Example 8

In example 2, the compound A was replaced by the compounds B and C, respectively, to obtain the medicament compositions. These medicament compositions were shown improved solubility in water as same as of the medicament compositon 2.

Example 9

A compound A (2 g), HPC-L (2 g) and HP-55 (2 g) were dissolved in methylene chloride (200 ml) and HPC-L (2 g) and HP-55 (2 g) and the solution was spray-dried using spray-drying machine (inlet temperature 70 °C, outlet temperature 45 °C) to obtain the medicament composition 11.

Example 10

In example 9, HP-55 was replaced by EC, AEA, Eudragit E, Eudragit S, Eudragit RS and Eudragit L, respectively, to obtain the medicament compositions 12, 13, 14, 15, 16 and 17.

Example 11

In example 9, HPC-L (2 g) and HP-55 (2 g) were replaced by HPC-L (1 g) and TC-5 (1 g), respectively, to obtain the medicament composition 18.

Example 12

A compound A (2 g), HPC-L (1 g), TC-5 (1 g) and dehydrocholic acid (0.5 g) were dissolved in methylene chloride (200 ml) and HPC-L (1 g) and TC-5 (1 g) and dehydrocholic acid (0.5g) and the solution was spray-dried using spray-drying machine (inlet temperature 70 °C, outlet temperature 45 °C) to obtain the medicament composition 19.

Example 13

In example 12, TC-5 (1 g) and dehydrocholic acid (0.5 g) were replaced by dehydrocholic acid (1 g) to obtain the medicament composition 20.

Referential example 6

A compound A (2 g) and Eudragit E (2 g) dissolved in methylene chloride (200 ml) and the solution was spray-dried using spray-drying machine to obtain the control medicament 7.

Referential example 7

In referential example 6, Eudragit E were replaced by AEA, Eudragit L, Eudragit S and dehydrocholic acid, respectively and treated as same as of in ref. example 6 to obtain the control medicaments 8, 9, 10 and 11.

Referential example 8

In example 6, TC-5 was replaced by vinyl acetate resin (Tanabe Seiyaku Co., food additive) and sucrose

fatty acid ester (Daiichi Kogyo Seiyaku Co. DK-ester F160) to obtain the control medicaments 12 and 13.

Test example

Stability test for amorphous state of the medicament compositions

1) Test method:

Solubility of the medicament compound in water at 37°C for 0 - 180 minutes is measured immediately after preparation of the medicament composition or the control medicament of the present invention. Area under the solubility curve at 0 - 180 min. is calculated from the thus obtained solubility curve, then the aria is set as X.

Solubility of the test sample in water at 37 °C for 0 -180 minutes is measured after storage under severe condition at 40 °C and 75 % R. H. for 4 weeks. The area under the solubility curve is set as Y.

Ratio of Y for X is calculated and the value obtained is estimated as the stability of amorphous state of a compound of the test sample. A value closer to 1 indicates the main-taining the amorphous state, and a value as smaller and smaller indicates the decease in solubility in water by recrystallization of the compound.

2) Test result:

Result is shown in Table 1.

Table 1

| Medicament composition | Stability ratio |
|---|---|
| 2 | 0.52 |
| 11 | 0.91 |
| 12 | 0.83 |
| 13 | 0.91 |
| 14 | 0.79 |
| 15 | 0.85 |
| 16 | 0.79 |
| 17 | 0.81 |
| 18 | 0.85 |
| 19 | 0.93 |
| Control | Stability ratio |
| 12 | 0.46 |
| 13 | 0.25 |

As a result hereinabove, the medicament compositions 11 - 19 are shown in maintaining the stability for

amorphous state under the severe condition at 40 °C and 75 % R. H. for 4 weeks strage as compared with the stability of the medicament composition 2 under the same condition.

## Claims

1. A composition which comprises an amorphous mixture of a compound of formula (I)

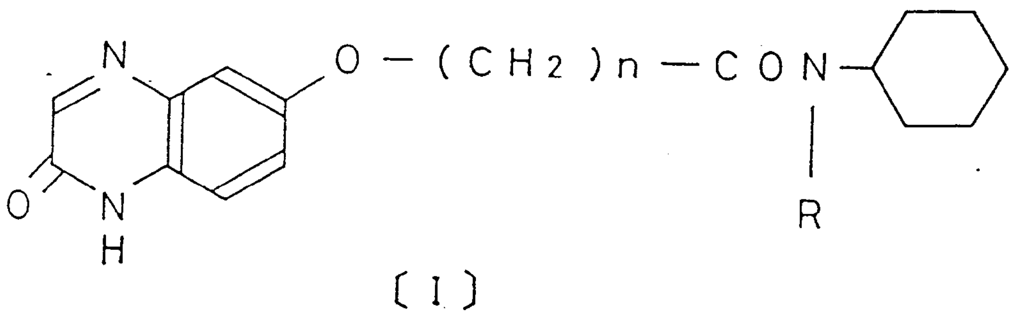

( I )

wherein R is methyl or ethyl and n is 3 or 4, and a hydroxypropyl cellulose, or the compound of formula (I) in amorphous form and a hydroxypropyl cellulose.

2. A composition according to claim 1 which comprises an amorphous mixture of the compound of formula (I), the hydroxypropyl cellulose and a stabilizing agent for an amorphous state.

3. A composition according to claim 2 wherein the stabilizing agent is a hydroxypropyl methyl cellulose, hydroxypropyl methyl cellulose phthalate, ethyl cellulose, aminoalkyl methacrylate copolymer, methacrylate copolymer, polyvinyl acetal diethylamino acetate or bile acid or a non-toxic salt thereof.

4. A process for the production of a composition as defined in any one of the preceding claims, which comprises adding the hydroxypropyl cellulose into the compound of formula (I) in the amorphous state or converting a mixture of the compound of formula (I) and hydroxypropyl cellulose to the amorphous state.

5. A process according to claim 4 wherein the compound of formula (I) in the amorphous state is obtained by drying a solution of the compound of formula (I) dissolved in an organic solvent.

6. A process according to claim 4 which comprises drying a solution of the compound of formula (I), hydroxypropyl cellulose and, if desired, stabilizing agent dissolved in an organic solvent.

7. A process according to claim 5 to 6 wherein the drying is spray-drying or fluidized bed granulation drying.

8. A composition as defined in any one of claims 1 to 3 for use in a method of treatment of the human or animal body by therapy.

9. A composition as defined in any one of claims 1 to 3 for use in a method of treatment of thrombosis or for use in improving the blood circulation.

10. Use of a composition as defined in any one of claims 1 to 3 in the manufacture of a medicament for the treatment of thrombosis or for improving the blood circulation.

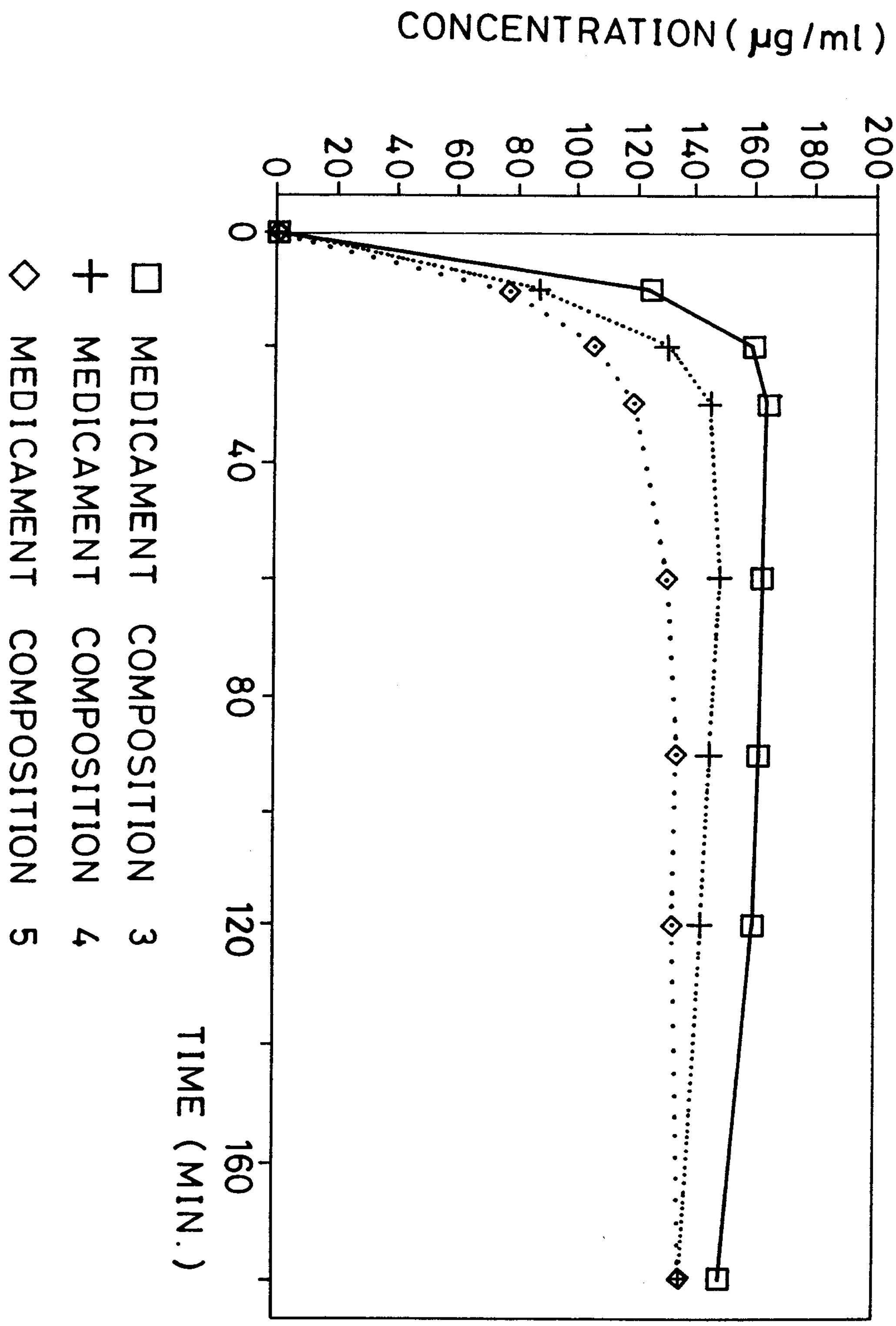
CONCENTRATION ( µg / ml )
0
20
40
60
80
100
120
140
160
180
200
0
40
80
120
160
TIME ( MIN. )
□ MEDICAMENT COMPOSITION 3
+ MEDICAMENT COMPOSITION 4
◇ MEDICAMENT COMPOSITION 5

FIG. 1

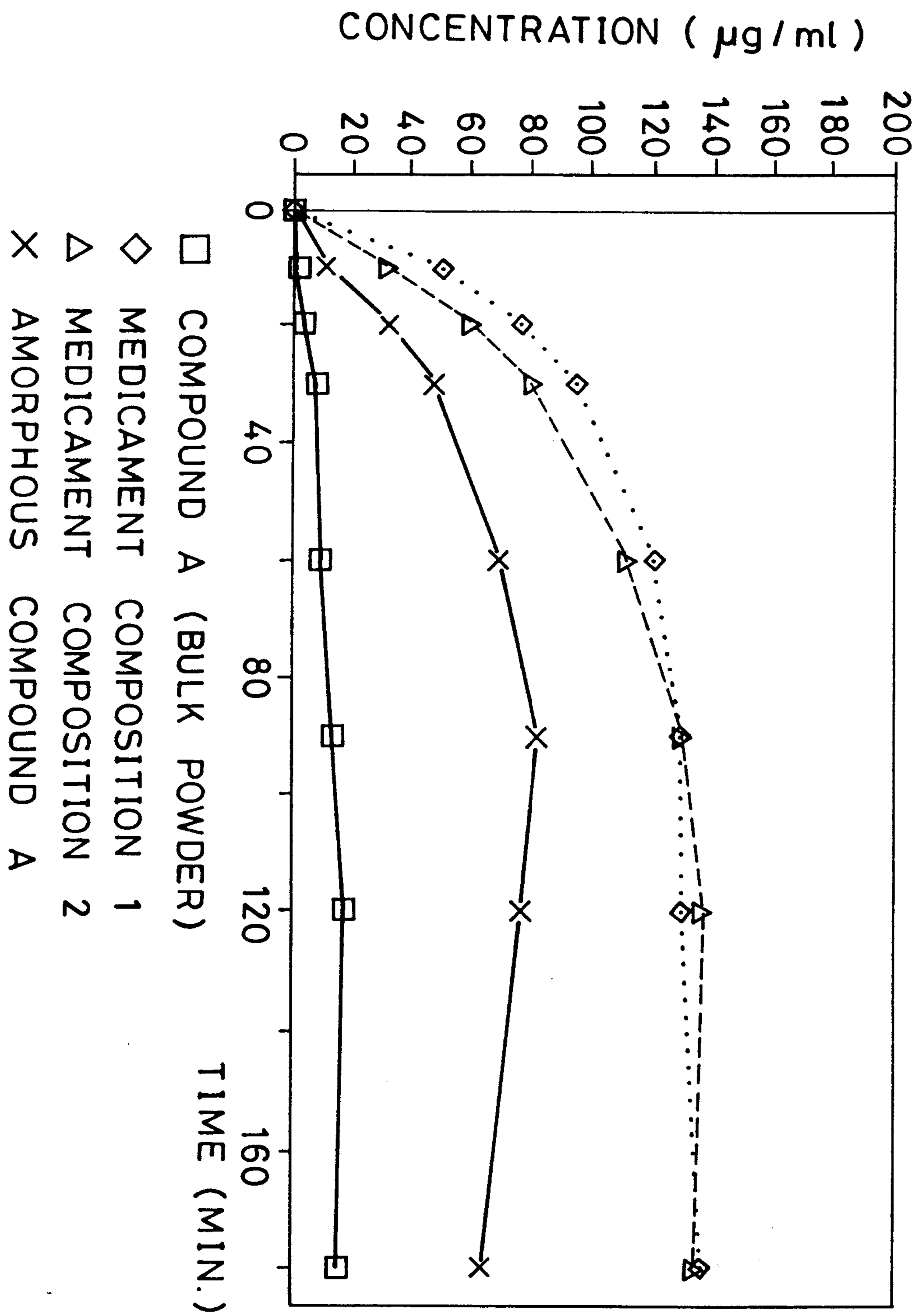
CONCENTRATION ( μg / ml )
0
20
40
60
80
100
120
140
160
180
200
0
40
80
120
160
TIME (MIN.)
COMPOUND A (BULK POWDER)
MEDICAMENT COMPOSITION 1
MEDICAMENT COMPOSITION 2
AMORPHOUS COMPOUND A

FIG. 2

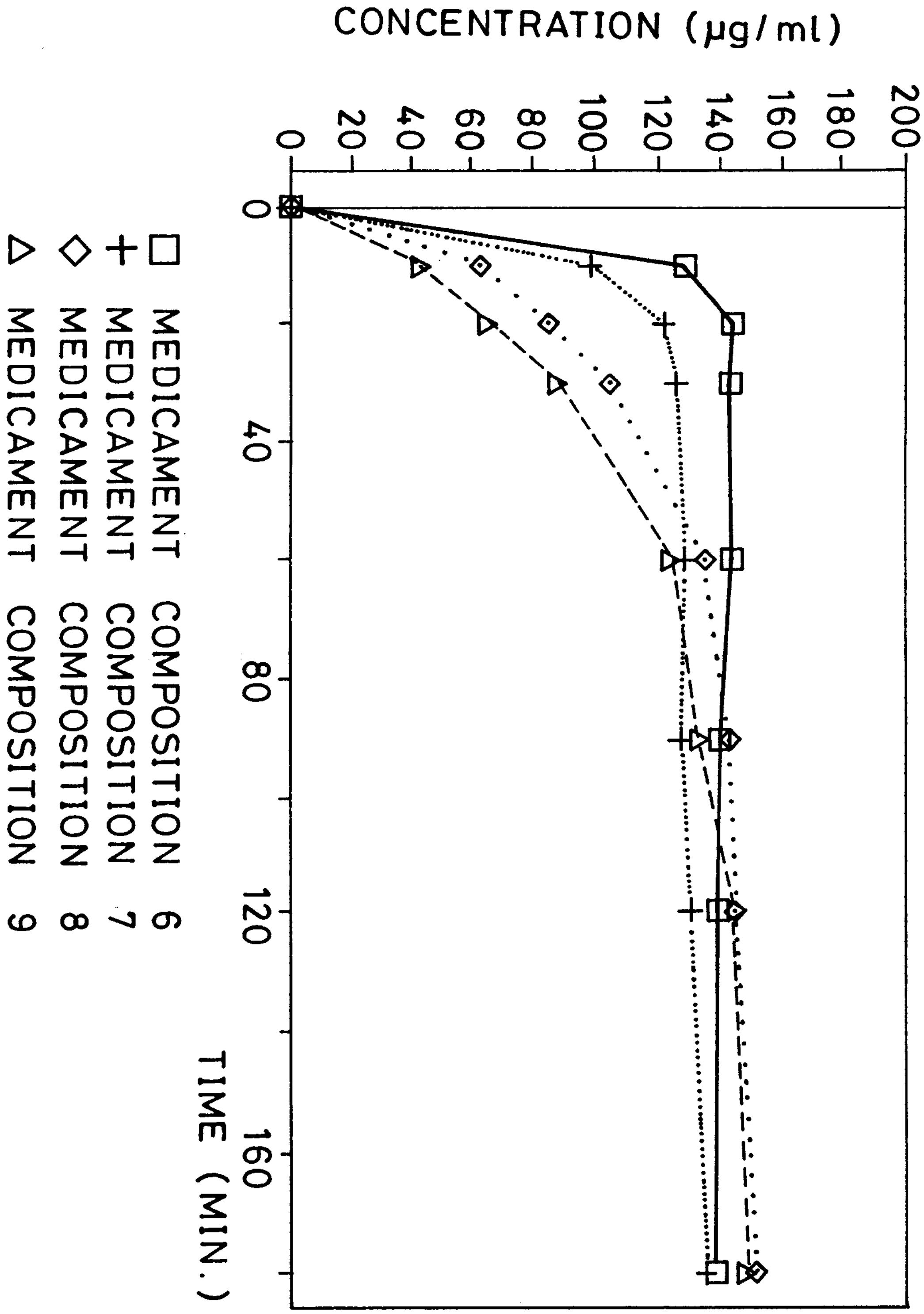
CONCENTRATION (µg/ml)
0
20
40
60
80
100
120
140
160
180
200
0
40
80
120
160
TIME (MIN.)
MEDICAMENT COMPOSITION 6
MEDICAMENT COMPOSITION 7
MEDICAMENT COMPOSITION 8
MEDICAMENT COMPOSITION 9

FIG. 3

FIG. 4

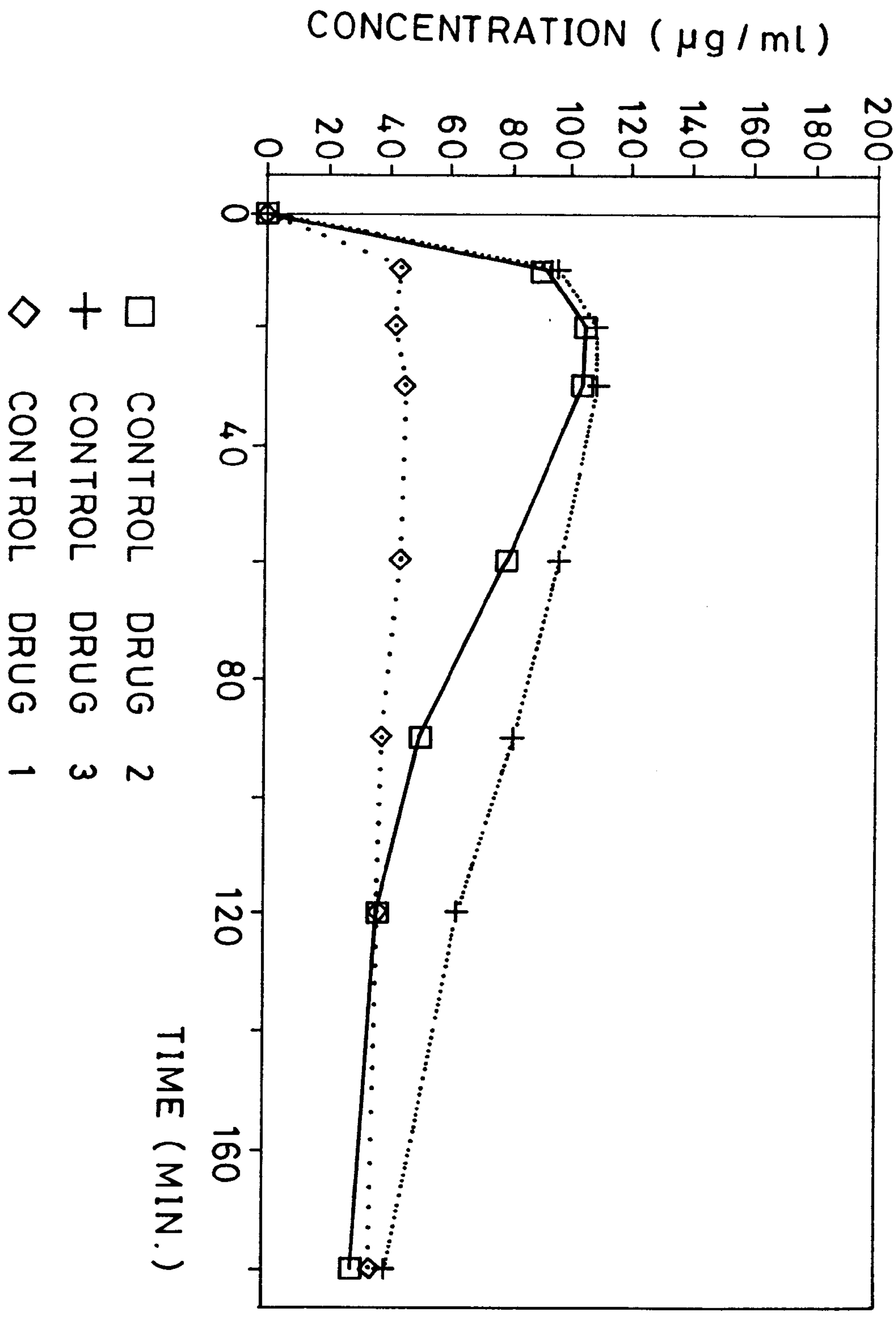
CONCENTRATION ( µg / ml )
0
20
40
60
80
100
120
140
160
180
200
0
40
80
120
160
TIME (MIN.)
CONTROL DRUG 2
CONTROL DRUG 3
CONTROL DRUG 1

FIG. 5

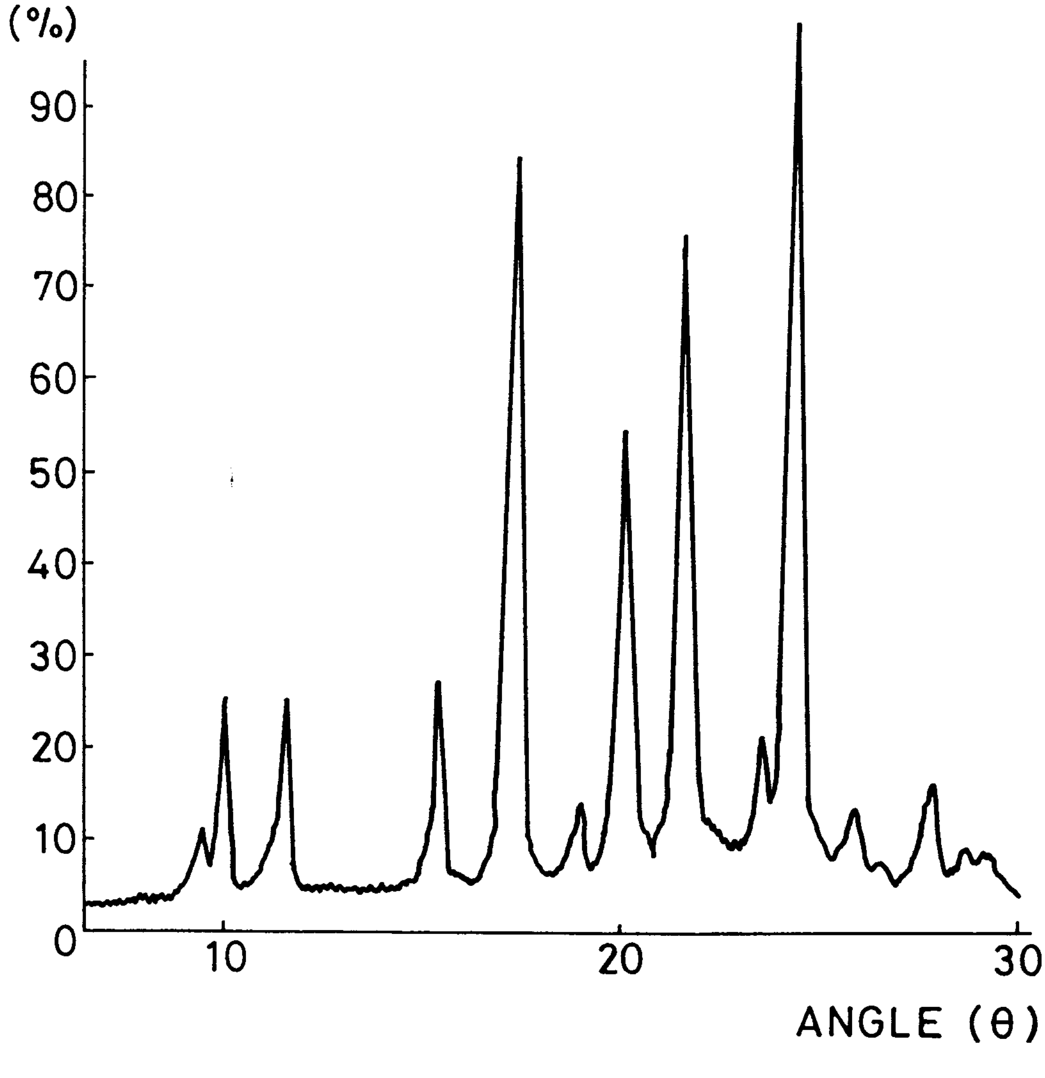

FIG. 6

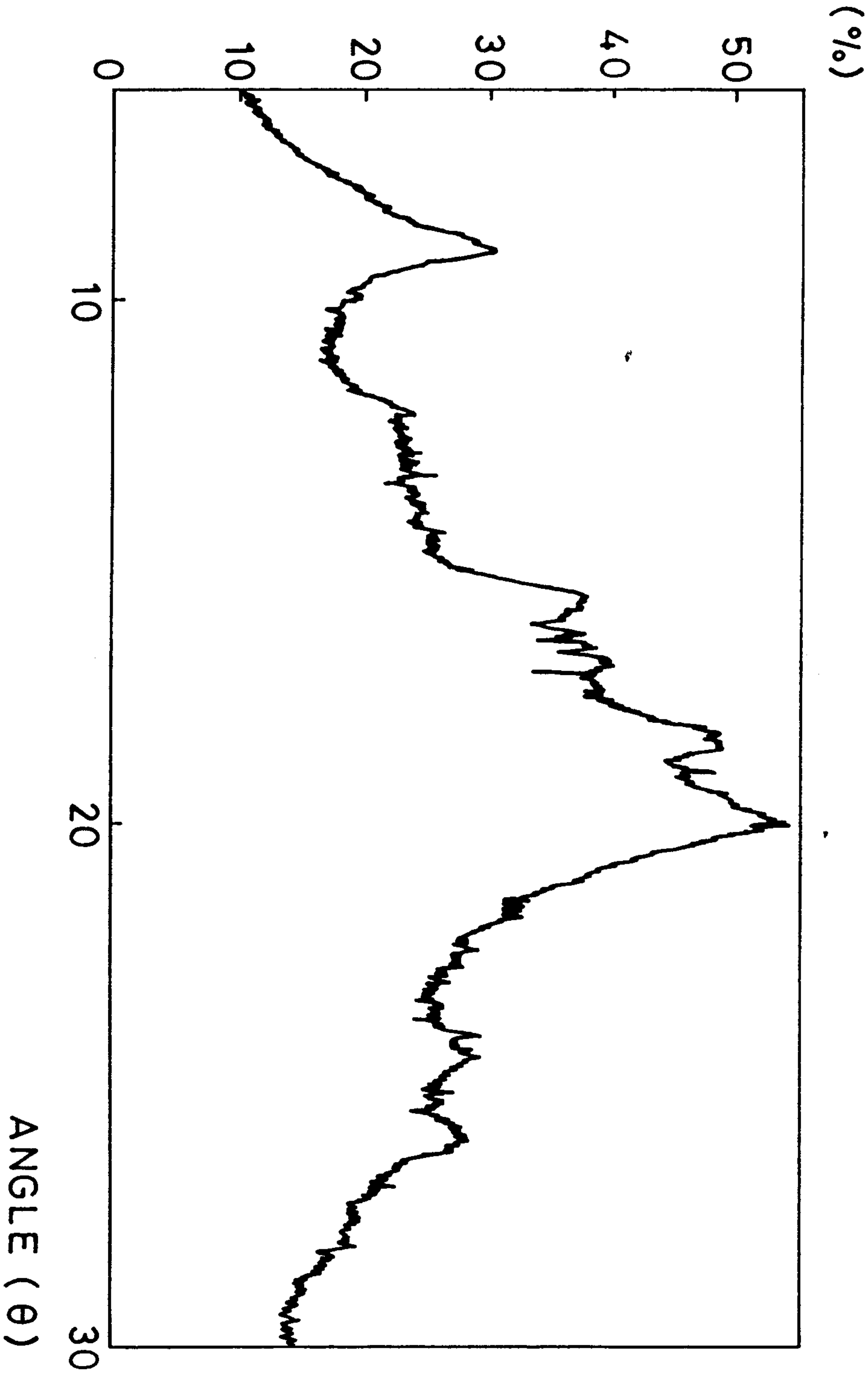

FIG. 7

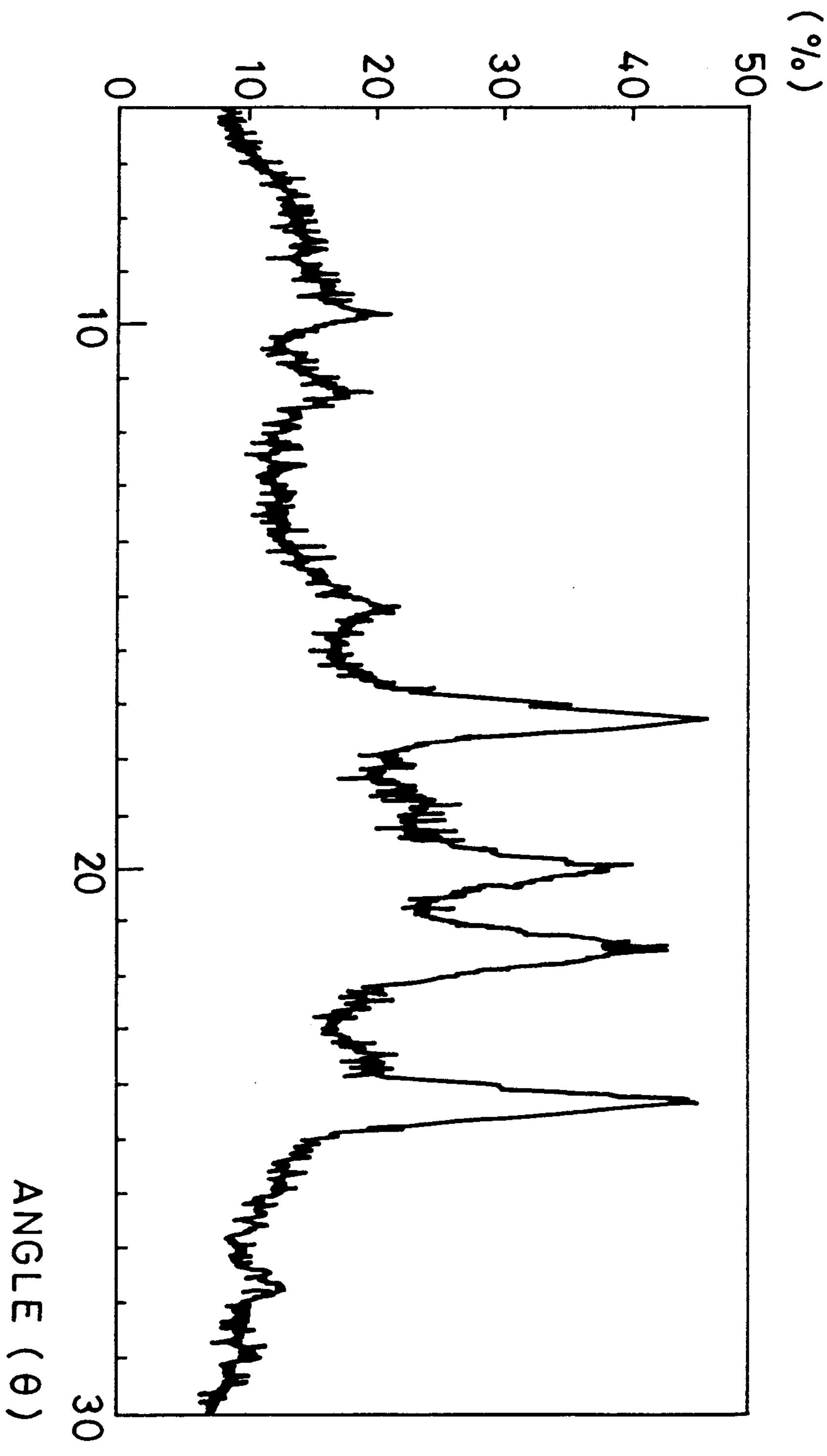

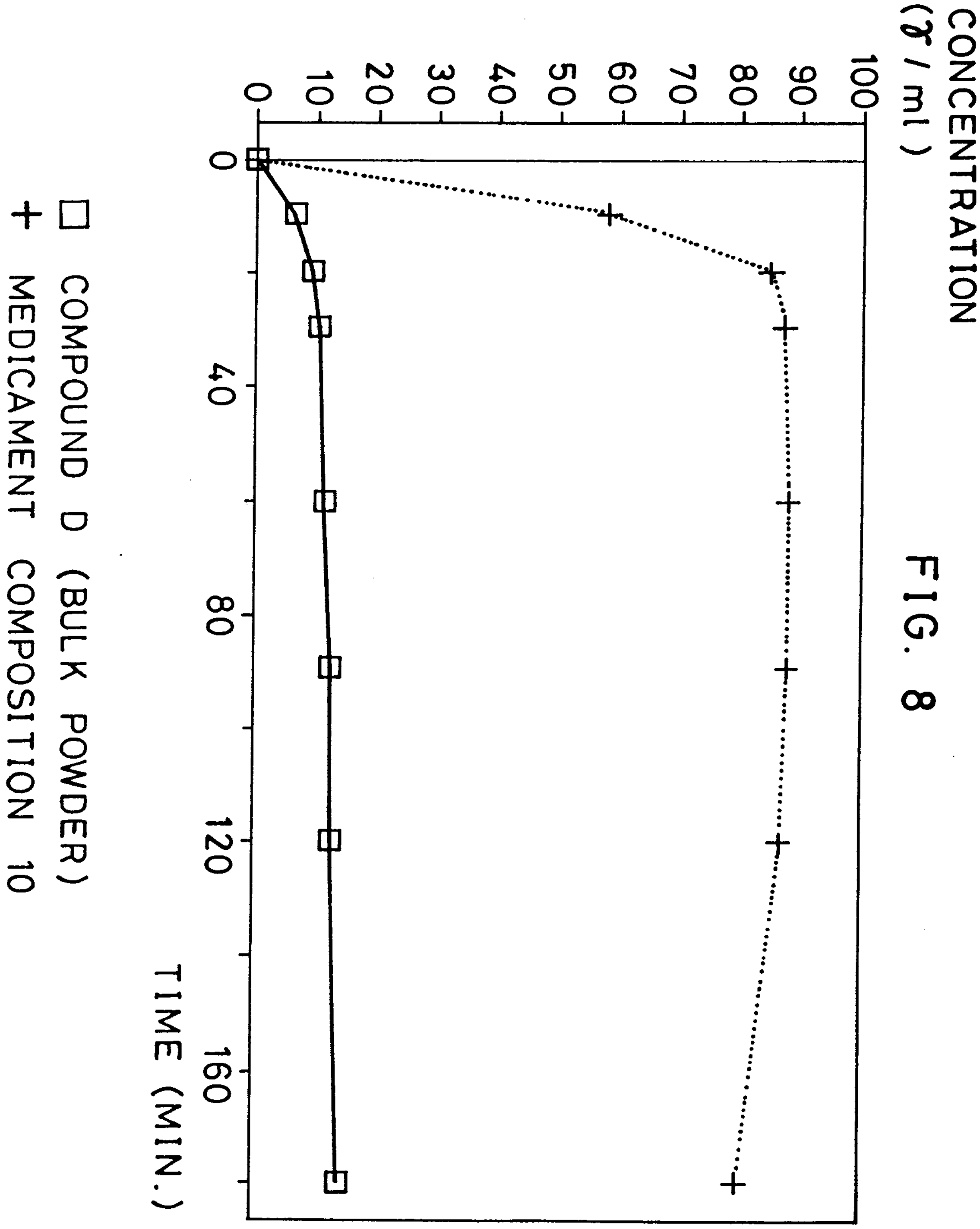
CONCENTRATION
(γ / ml )
100
90
80
70
60
50
40
30
20
10
0
0
40
80
120
160
TIME (MIN.)
□ COMPOUND D (BULK POWDER)
+ MEDICAMENT COMPOSITION 10

FIG. 8

FIG. 9

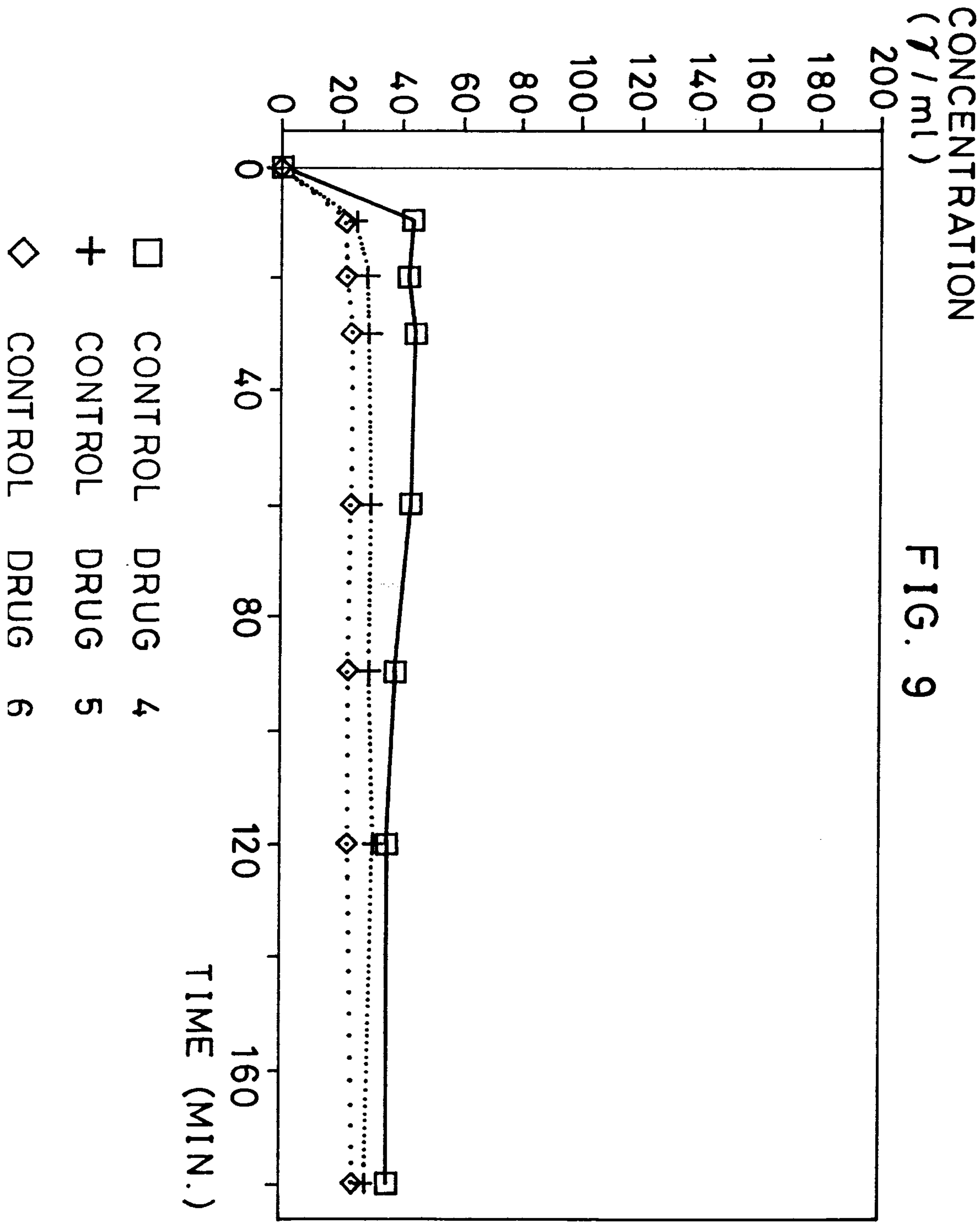
CONCENTRATION
( γ / ml )
200
180
160
140
120
100
80
60
40
20
0
0
40
80
120
160
TIME (MIN.)
CONTROL DRUG 4
CONTROL DRUG 5
CONTROL DRUG 6

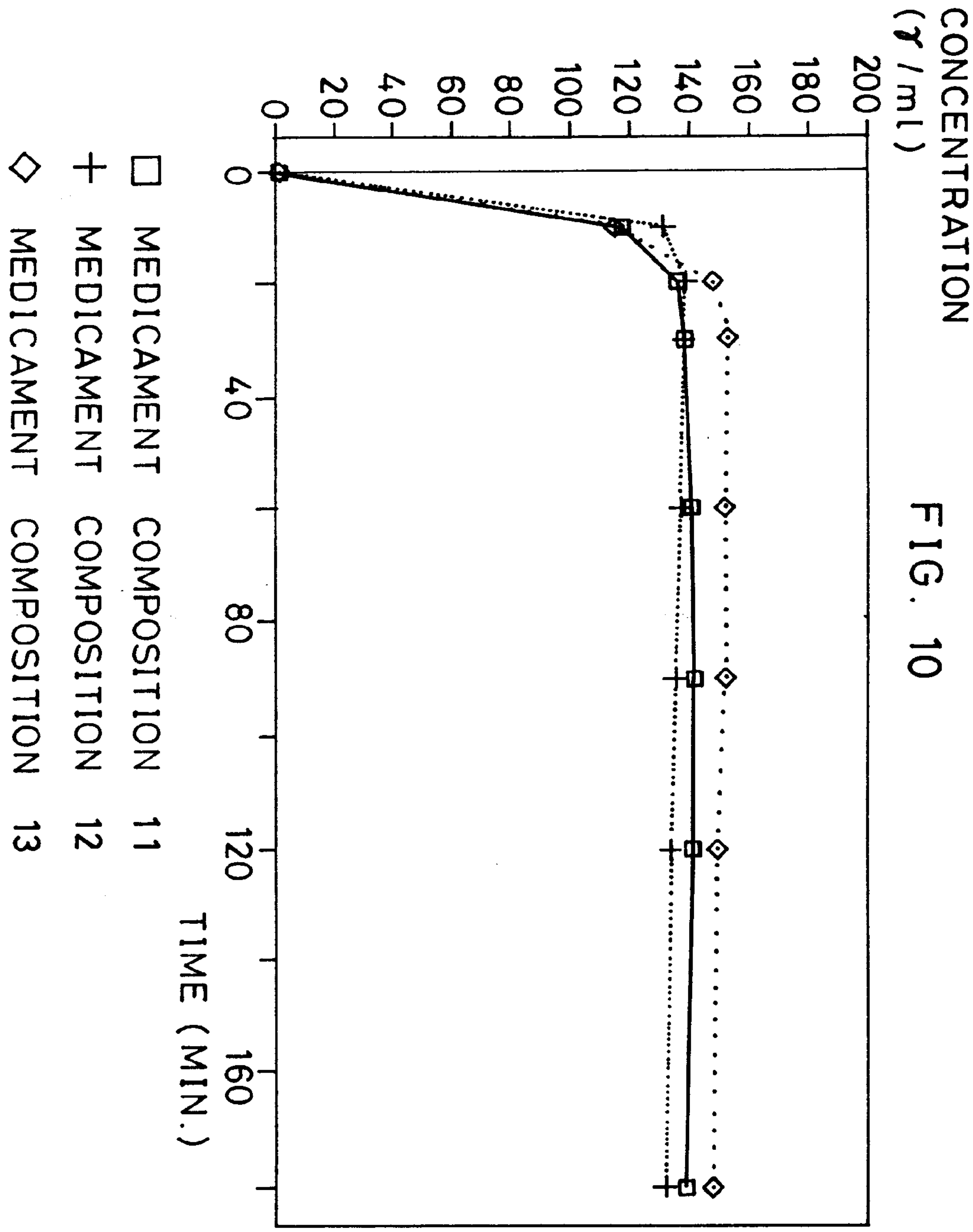
CONCENTRATION
(γ / ml)
0
20
40
60
80
100
120
140
160
180
200
0
40
80
120
160
TIME (MIN.)
MEDICAMENT COMPOSITION 11
MEDICAMENT COMPOSITION 12
MEDICAMENT COMPOSITION 13

FIG. 10

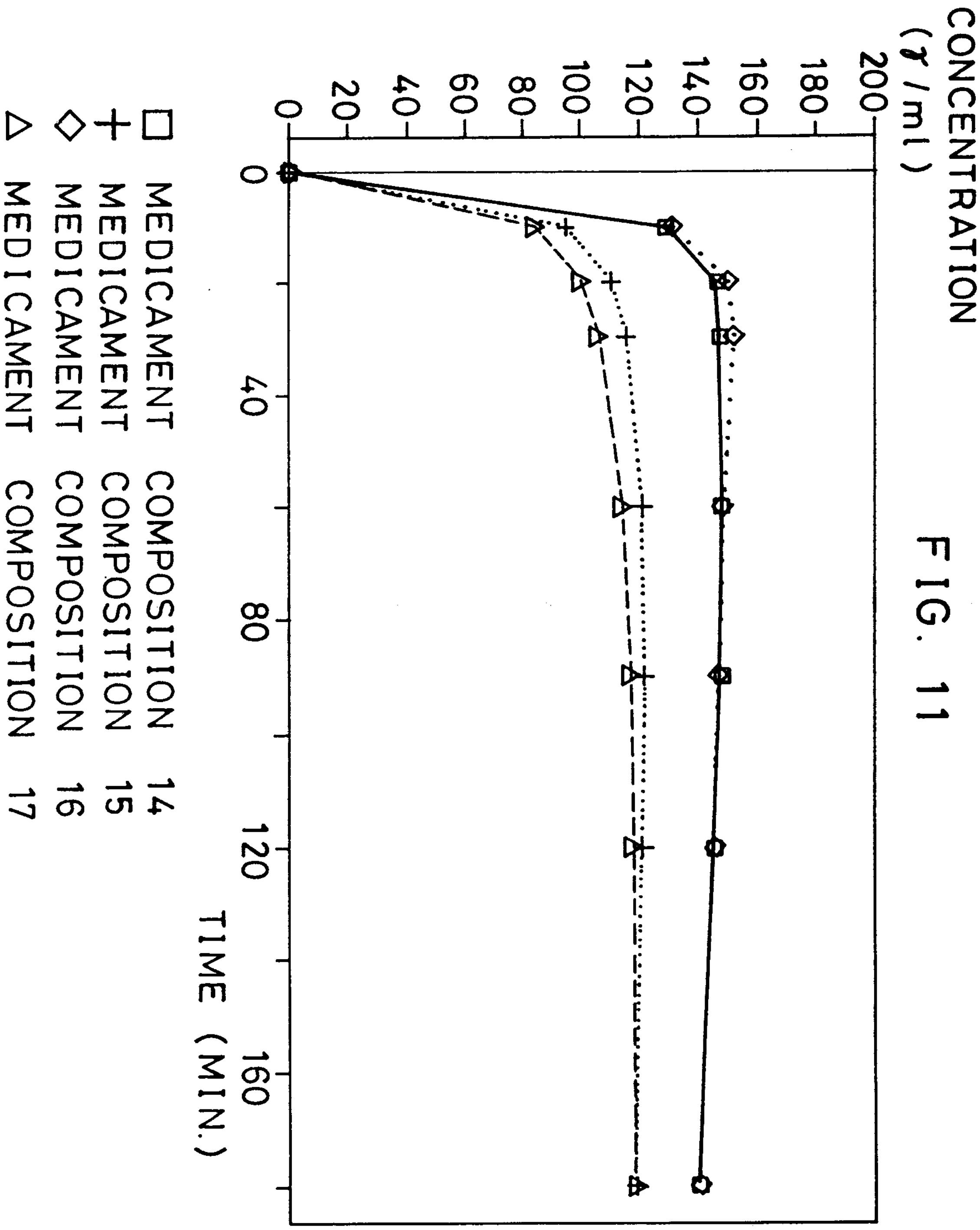
CONCENTRATION
(γ/ml)
200
180
160
140
120
100
80
60
40
20
0
0
40
80
120
160
TIME (MIN.)
□ MEDICAMENT COMPOSITION 14
+ MEDICAMENT COMPOSITION 15
◇ MEDICAMENT COMPOSITION 16
△ MEDICAMENT COMPOSITION 17

FIG. 11

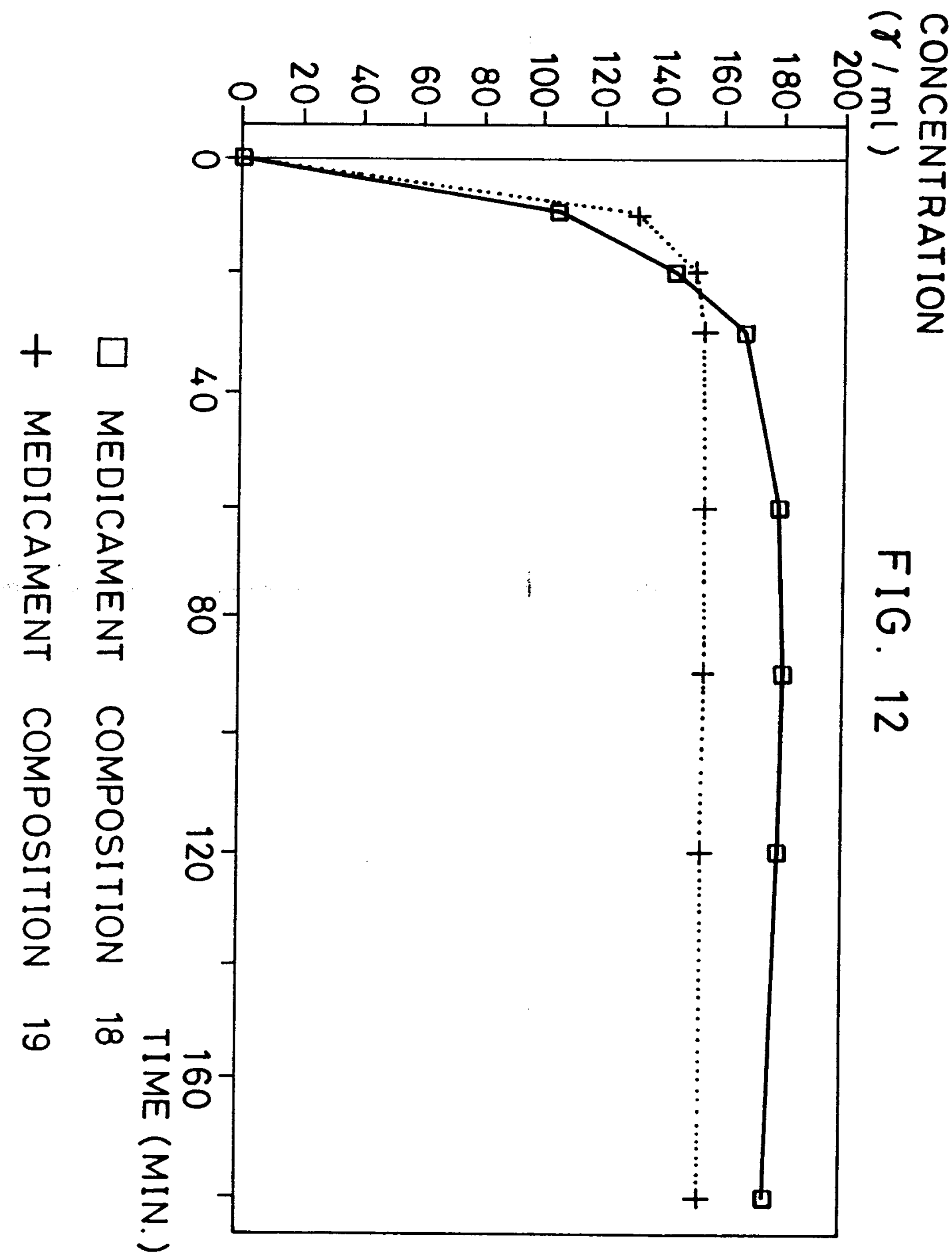
CONCENTRATION
(γ / ml)
200
180
160
140
120
100
80
60
40
20
0
0
40
80
120
160
TIME (MIN.)
□ MEDICAMENT COMPOSITION 18
+ MEDICAMENT COMPOSITION 19

FIG. 12

FIG. 13

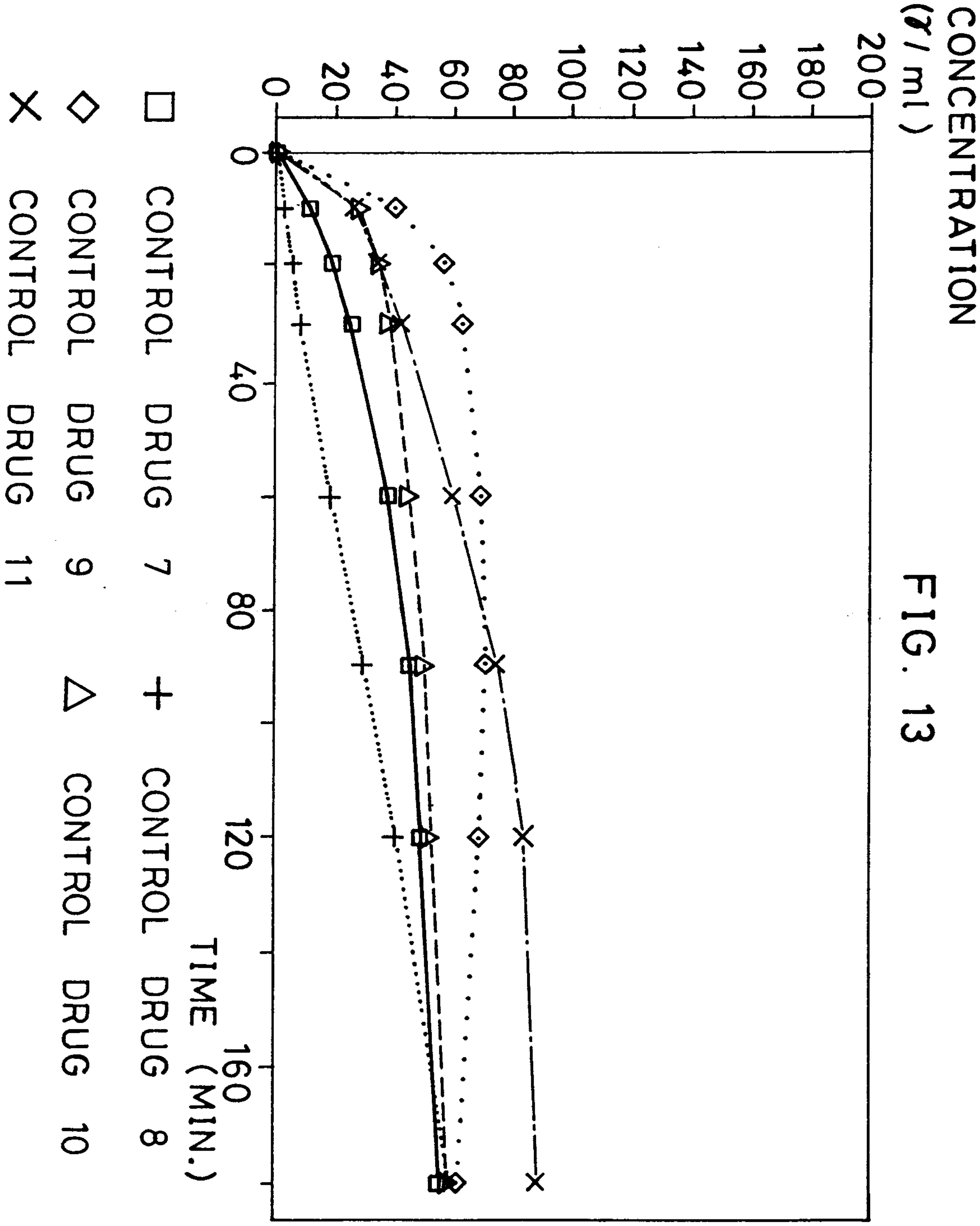
CONCENTRATION
(γ/ml)
200
180
160
140
120
100
80
60
40
20
0
0
40
80
120
160
TIME (MIN.)
CONTROL DRUG 7
CONTROL DRUG 8
CONTROL DRUG 9
CONTROL DRUG 10
CONTROL DRUG 11

FIG. 14

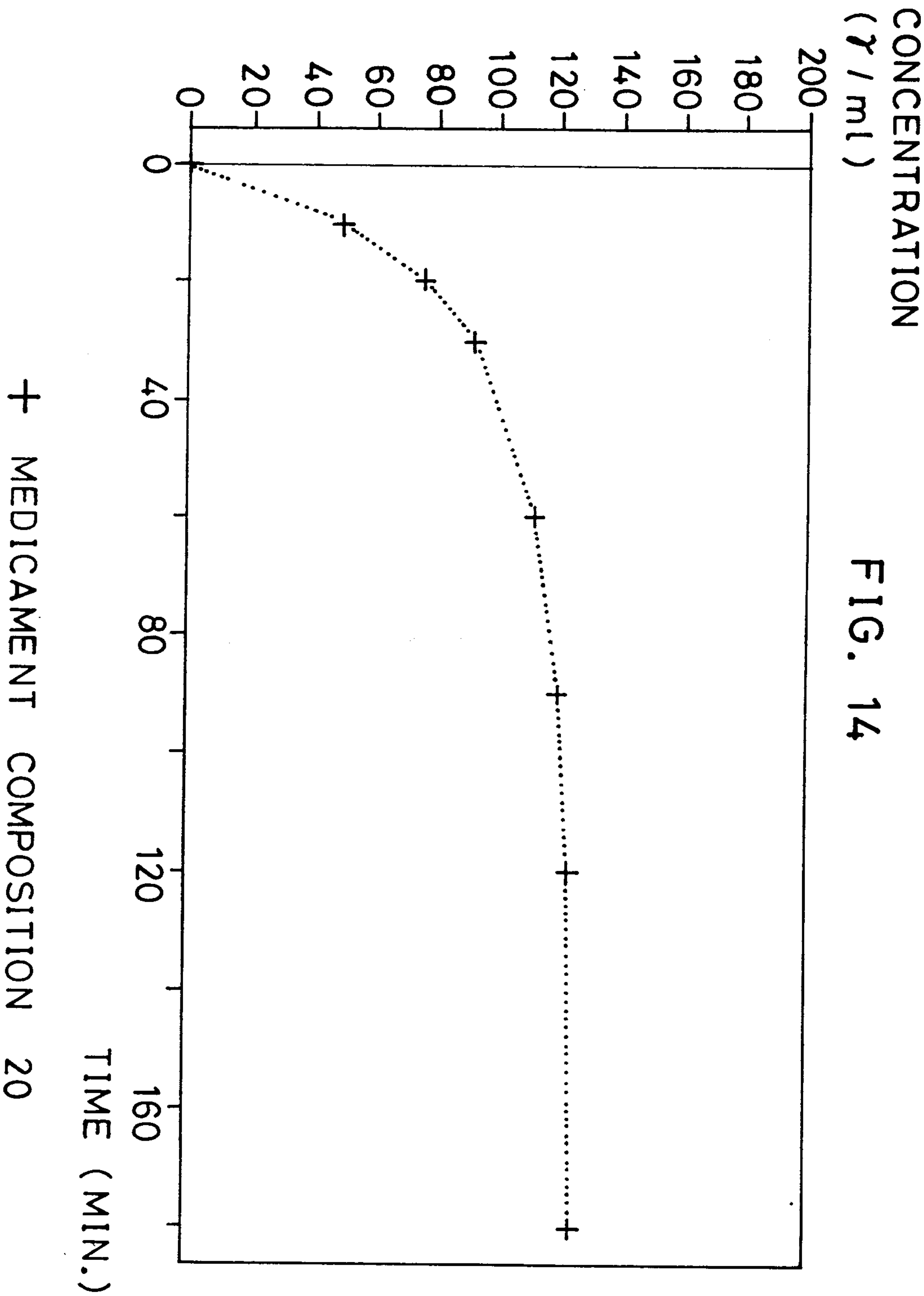
CONCENTRATION
( γ / ml )
0
20
40
60
80
100
120
140
160
180
200
0
40
80
120
160
TIME (MIN.)
+ MEDICAMENT COMPOSITION 20

European Patent Office

# EUROPEAN SEARCH REPORT

Application Number

EP 91 30 9891

| Category | Citation of document with indication, where appropriate, of relevant passages | Relevant to claim | CLASSIFICATION OF THE APPLICATION (Int. Cl.5) |
|---|---|---|---|
| A | STN INTERNATIONAL INFORMATION SERVICES, DATA BASE: CHEMICAL ABSTRACTS, vol. 114, no. 20, abstract no. 192605t, Columbus, Ohio, US; & JP-A-02 221 223 (TOYO JOZO CO., LTD) 04-09-1990 * Abstract * | 1,6,8-10 | A 61 K 9/14<br>A 61 K 9/18<br>A 61 K 47/38<br>A 61 K 31/495 |
| D,A | DATABASE WPIL, accession no. 90-096555, Derwent Publications Ltd, London, GB; & JP-A-2 049 720 (MITSUBISHI KASEI CORP.) 20-02-1990 * Abstract * | 1,6-7 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 17-12-1991 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)